# EUROPEAN PATENT SPECIFICATION

(11) **EP 3 063 124 B1**
(45) Date of publication and mention of the grant of the patent: **22.11.2017**
(21) Application number: 13786877.4
(22) Date of filing: 30.10.2013
(51) Int. Cl.: C07C 213/06, C11D 1/62, C12P 13/00

(54) **BETAINE ESTERS AND PROCESS FOR MAKING AND USING**
BETAINESTER UND VERFAHREN ZUR HERSTELLUNG UND VERWENDUNG
ESTERS DE BÉTAÏNE ET LEUR PROCÉDÉ DE FABRICATION ET D'UTILISATION

(43) Date of publication of application: 07.09.2016
(73) Proprietor: Eastman Chemical Company, Kingsport, TN 37660 (US)
(72) Inventor: BURK, Christopher Harlan, Gray, Tennessee 37615 (US); CLENDENNEN, Stephanie Kay, Kingsport, Tennessee 37663 (US); BOAZ, Neil Warren, Kingsport, Tennessee 37663 (US)
(74) Representative: Ricker, Mathias
(86) International application number: PCT/US2013/067410
(87) International publication number: WO 2015/065354

(56) References cited:
- WO-A1-2012/148739
- US-B1- 7 667 067
- MILLER C ET AL: "CHARACTERISTICS OF AN IMMOBILIZED LIPASE FOR THE COMMERCIAL SYNTHESIS OF ESTERS", JOURNAL OF THE AMERICAN OIL CHEMISTS' SOCIETY, SPRINGER, DE, vol. 65, no. 6, 1 June 1988 (1988-06-01), pages 927-931, XP001161185, ISSN: 0003-021X, DOI: 10.1007/BF02544512
- GANDHI N N: "APPLICATIONS OF LIPASE", JOURNAL OF THE AMERICAN OIL CHEMISTS' SOCIETY, SPRINGER, DE, vol. 74, no. 6, 1 June 1997 (1997-06-01), pages 621-634, XP000693786, ISSN: 0003-021X, DOI: 10.1007/S11746-997-0194-X

## Description

### FIELD OF THE INVENTION

This invention pertains to betaine esters and processes for the preparation and use thereof.

### BACKGROUND OF THE INVENTION

There is an increasing industrial and societal need for the preparation of ingredients that reduce or eliminate organic solvents and irritants, employ reagents that are themselves biocompatible and that optimally use starting materials derived from a natural source or are "nature-equivalent." This is of urgent interest in consumer-facing industries such as personal and household care. One class of materials that might be approached in a "greener" manner is surfactants. In particular, there is a need for new betaines that are made in a more environmentally-friendly manner. Betaines are zwitterionic surfactants used in the personal care, household care, and other industries. They are classified as specialty co-surfactants that complement the performance of the primary surfactants. These co-surfactants also increase the mildness of the formulation by reducing irritation associated with purely ionic surfactants.

Betaines are commonly produced by a multi-step process based on coconut or palm kernel oil. For example, one process for the preparation of a prototypical betaine, fatty acid amidopropyl betaine, involves the amidation of fatty acids with 3-dimethylaminopropylamine (DMAPA) at high temperatures (150-175°C). The intermediate fatty aminoamide is then reacted with sodium chloroacetate to afford the final product. The amidation requires high temperatures for conversion and distillation to remove unreacted starting materials. These high reaction temperatures can generate by-products and impart color to the products, requiring additional steps to remove the by-products and the color. DMAPA is also a known sensitizer and is found in trace quantities in the final formulation. Thus, betaines prepared under mild conditions without the use of DMAPA would be of great interest.

It would be highly desirable for the production of the betaines to occur under mild conditions and in high yield. Such a process would take place at lower temperatures, with fewer processing steps and by-products and it would lessen environmental impacts. These objectives can be met, for example, by the direct esterification process disclosed below, with the production of the betaines occuring directly from the fatty acids. The direct esterification process is a significant improvement over the transesterfication process as it avoids a process step and eliminates the use of an alcohol such as methanol and its required recycle.

WO 2012/148739 A1 discloses betaine esters of formula 1, 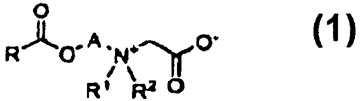 including dialkylaminoalkyl cocoate betaines, wherein R is selected from the group consisting of C₁-C₂₂ hydrocarbyl, C₃-C₈ cycloalkyl, C₆-C₂₀ carbocyclic aryl, and C₄-C₂₀ heterocyclic wherein the heteroatoms are selected from the group consisting of sulfur, nitrogen, oxygen, and mixtures thereof;
R¹ and R² are the same or are independently selected from the group consisting of C₁-C₆ alkyl, C₂-C₆ alkenyl, C₄-C₆ dienyl, and C₃-C₈ cycloalkyl; and
A is selected from the group consisting of C₁-C₁₀ divalent hydrocarbyl, C₃-C₈ cycloalkylene, C₆-C₁₀ carbocyclic arylene, and C₄-C₁₀ divalent heterocyclic wherein the heteroatoms are selected from sulfur, nitrogen, and oxygen.

### BRIEF SUMMARY OF THE INVENTION

In accordance with one aspect of the invention there is provided a direct esterification process for the preparation of betaine, represented by the general formula 1, 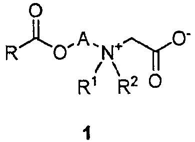 comprising:
a) reacting a carboxylic acid 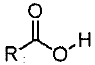 wherein R selected from the group consisting of of C₅-C₁₇ alkyl, C₅-C₁₇ alkenyl, C₅-C₁₇ dienyl, C₅-C₁₇ trienyl, and mixtures thereof, and R⁶ is a C₁-C₆ alkyl;
   with a dialkylamino alcohol **3:** 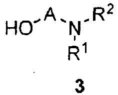 in the presence of an enzyme to form an intermediate **4:** 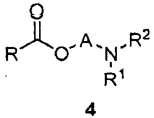 wherein R¹ is methyl and R² is selected from the group consisting of C₁-C₅ alkyl;
   A is selected from the group consisting of C₃-C₁₀ alkylene and C₃-C₁₀ and alkenylene; and
b) reacting intermediate **4** with sodium chloroacetate to produce a betaine, wherein the enzyme is Candida antarctica lipase B immobilized on acrylic resin or Candida antarctica lipase B immobilized on a porous fluoropolymer, as defined in claim 1.

Further aspects of the invention are set out below and in the claims hereinafter.

### DETAILED DESCRIPTION

An general embodiment of the invention is a direct esterification process for the preparation of betaine, represented by the general formula 1, 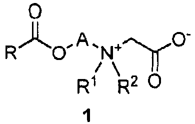 comprising:
a) reacting a carboxylic acid 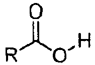 wherein R selected from the group consisting of of C₅-C₁₇ alkyl, C₅-C₁₇ alkenyl, C₅-C₁₇ dienyl, C₅-C₁₇ trienyl, and mixtures thereof, and R⁶ is a C₁-C₆ alkyl;
   with a dialkylamino alcohol **3:** 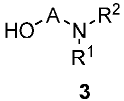 in the presence of an enzyme to form an intermediate **4:** 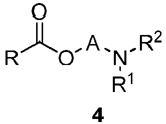 wherein R¹ is methyl and R² is selected from the group consisting of C₁-C₅ alkyl;
   A is selected from the group consisting of C₃-C₁₀ alkylene and C₃-C₁₀ and alkenylene; and
b) reacting intermediate **4** with sodium chloroacetate to produce a betaine, wherein the enzyme is Candida antarctica lipase B immobilized on acrylic resin or Candida antarctica lipase B immobilized on a porous fluoropolymer.
In one aspect of the direct esterification process, R is selected from C₅-C₁₇ alkyl and mixtures thereof; R¹ is methyl and R² is selected from C₁-C₅ alkyl, and A is selected from C₃-C₈ alkylene and C₃-C₈ alkenylene. It has been found by experimentation that when moiety A has more than 2 carbon atoms significantly better conversion rates are obtained (see Examples). Hence the range claimed for moiety A is C₃-C₁₀ alkylene or alkenylene.

In another aspect, R² is methyl, and A is selected from C₃-C₈ alkylene and C₃-C₈ alkenylene. In yet another aspect, R² is methyl and A is 1,3-propylene.

The first step of the direct esterification process can be carried out without solvent or in an inert solvent chosen from cyclic or acyclic ether solvents such as diethyl ether, diisopropyl ether, tert-butyl methyl ether, or tetrahydrofuran, aromatic hydrocarbons such as benzene, toluene, or xylene, aliphatic or alicyclic saturated or unsaturated hydrocarbons such as hexane, heptane, cyclohexane, or limonene, halogenated hydrocarbons such as dichloromethane, dichloroethane, dibromoethane, tetrachloroethylene, or chlorobenzene, polar aprotic solvents such as acetonitrile, dimethyl formamide, or dimethyl sulfoxide, or mixtures thereof.

In an aspect of the invention no solvent is used, thereby reducing the need to handle, supply and dispose of the solvents.

In a preferred aspect of the invention an inert solvent, such as heptane is used in the direct esterification process. The inert solvent is preferably an azeotroping agent which facilitates removal of water from the reaction mixture and drives the reaction to higher conversions. In embodiments of the invention conversion rates above 98% and above 99% are possible (for which see the examples hereinafter).

The present invention allows the process to be conducted without an active solvent such as an alcohol (typically methanol) thereby obviating the need to subsequently remove the solvent alcohol from the product.

The first step of the direct esterification process may be carried out at a temperature from about -100 °C to about the boiling point of the solvent, from about 20 to about 80 °C, or from about 50 to about 70 °C. The amount of dialkylamino alcohol 3 may be from about 0.85 to about 20 equivalents based on the carboxylic acid, or can be from about 1 to about 10 equivalents, or even from about 1 to about 1.5 equivalents.

The enzyme used in the first step of the direct esterification process is chosen from Novozym™ 435 (Candida antarctica lipase B immobilized on acrylic resin) or Candida antarctica lipase B immobilized on a porous fluoropolymer support as described in US Patent Pub 2012/0040395.

Removal of the water byproducts can be done chemically via a water absorbent (e.g., molecular sieves) or by physical removal of the water.

According to a further aspect of the invention, this by-product removal can be done by evaporation, either by purging the reaction mixture with an inert gas such as nitrogen, argon, or helium, or by performing the reaction at reduced pressures, or both. These conditions can afford >98% conversion of the carboxylic acid to intermediate **4.**

Thus according to preferred embodiments, pressure for the reaction is from about 0.13 kPa (1 torr) to about ambient pressure, or from about 6.7 kPa (50 torr) to about ambient pressure. Any organic solvent that is included in this process may or may not be removed along with the water. The organic solvent used may assist in removal of the water byproduct by azeotropic distillation. Preferred examples of dialkylamino alcohol **3** include dimethylaminopropanol.

The second step of the direct esterification process to generate the final product **1** comprises the reaction of intermediate **4** with sodium chloroacetate. The second step of the direct esterification process can be carried out without solvent or in an inert solvent chosen from water, cyclic or acyclic alcohol solvents such as methanol, ethanol, 1-propanol, 2-propanol, 1-butanol, 2-butanol, isobutanol, ethylene glycol, 1,2-propanediol, or 1,3-propanediol, cyclic or acyclic ether solvents such as diethyl ether, diisopropyl ether, tert-butyl methyl ether, or tetrahydrofuran, aromatic hydrocarbons such as benzene, toluene, or xylene, aliphatic or alicyclic saturated or unsaturated hydrocarbons such as hexane, heptane, cyclohexane, or limonene, halogenated hydrocarbons such as dichloromethane, dichloroethane, dibromoethane, tetrachloroethylene, or chlorobenzene, polar aprotic solvents such as acetonitrile, dimethyl formamide, or dimethyl sulfoxide, or mixtures thereof.

The second step of direct esterification process may be carried out at a temperature of from about -100 °C to about the boiling point of the solvent, from about 25 to about 150 °C, or from about 50 to about 100 °C. The amount of sodium chloroacetate may be from about 0.75 to about 20 equivalents based on the amount of intermediate **4,** from about 1 to about 10 equivalents, or from about 1 to about 1.5 equivalents. If included, a base is chosen from metal hydroxides or metal carbonates. According to an embodiment, bases can be sodium hydroxide and potassium hydroxide. The amount of base can be from about 0 molar equivalents to about 1 molar equivalent based on intermediate **4** or in an amount high enough to keep the reaction mixture basic, for example at about pH 8-9.

The intermediate **4** and the product **1** of the process may be isolated using methods known to those of skill in the art, e.g., extraction, filtration, or crystallization.

The betaine esters are molecules possessing both hydrophilic and hydrophobic regions, making them useful as surfactants in a number of formulated product applications, including personal care products such as skin care, hair care or other cosmetic products, household and industrial surface cleaners, disinfectants, metal working, rust inhibitors, lubricants, agrochemicals, and dye dispersions. Betaines can also be used as emulsifiers and thickening agents in emulsions. Betaines are often formulated into products as secondary surface-active agents. Although a primary use is as humectants and foaming agents, betaines are also used for their anti-static and viscosity-controlling properties.

Such product formulations can contain from about 0.001 weight % to about 20 weight %, from about 0.01 weight % to about 15 weight %, or even from about 0.1 weight % to about 10 weight % of the betaine esters.

Product formulations made in accordance with the process of the invention may include other surfactants in addition to the betaine esters. These surfactants can include anionic surfactants (such as alcohol ether sulfates, linear alkylbenzene sulfonates, acyl isethionates), cationic surfactants (such as quaternary ammonium salts, fatty amine oxides, and ester quats), and non-ionic surfactants (such as alky polyglycosides, alcohol ethoxylates, and fatty alcanol amides). Such ingredients are known to those of skill in the art.

The cosmetic, skin, and hair care compositions may also contain other skin conditioning ingredients or cosmetically acceptable carriers in addition to the betaine esters.

Such formulations may also contain skin care ingredients/carriers such as retinol, retinyl esters, tetronic acid, tetronic acid derivatives, hydroquinone, kojic acid, gallic acid, arbutin, α-hydroxy acids, niacinamide, pyridoxine, ascorbic acid, vitamin E and derivatives, aloe, salicylic acid, benzoyl peroxide, witch hazel, caffeine, zinc pyrithione, and fatty acid esters of ascorbic acid. Such other ingredients are known to those of skill in the art.

Other ingredients that may be included in these formulations include conditioning agents (such as polyquaterniums and panthenol), pearlizing agents (such as glycol distearate, distearyl ether, and mica), UV filters (such as octocrylene, octyl methoxycinnamate, benzophenone-4, titanium dioxide, and zinc oxide), exfoliation additives (such as apricot seeds, walnut shells, polymer beads, and pumice), silicones (such as dimethicone cyclomethicone, and amodimethicone), moisturizing agents (such as petrolatum, sunflower oil, fatty alcohols, and shea butter), foam stabilizers (such as cocamide MEA and cocamide DEA), anti-bacterial agents such as triclosan, humectants such as glycerin, thickening agents (such as guar, sodium chloride, and carbomer), hair and skin damage repair agents (such as proteins, hydrolyzed proteins, and hydrolyzed collagen), and foam boosters such as cocamide MIPA. Such other ingredients are known to those of skill in the art.

Many preparations are known in the art, and include formulations containing acceptable carriers such as water, oils and/or alcohols and emollients such as olive oil, hydrocarbon oils and waxes, silicone oils, other vegetable, animal or marine fats or oils, glyceride derivatives, fatty acids or fatty acid esters or alcohols or alcohol ethers, lecithin, lanolin and derivatives, polyhydric alcohols or esters, wax esters, sterols, phospholipids and the like. These same general ingredients can be formulated into liquids (such as liquid soaps, shampoos, or body washes), creams, lotions, gels, or into solid sticks by utilization of different proportions of the ingredients and/or by inclusion of thickening agents such as gums or other forms of hydrophilic colloids.

Following is a description by way of example only of specific embodiments of the invention, along with comparative examples.

### Examples

### Comparative Example - (conventional transesterification)

### Comparative Example 1 (a)

### Preparation of Ethyl Cocoate

To a jar was added potassium hydroxide (2 g) and ethanol (72 g). The solution was stirred for 1 hour. To a separate jar was added coconut oil (200 g). The solid was heated to a melt and the KOH/EtOH solution was added and the mixture was stirred overnight. The mixture was transferred to a separation funnel and allowed to separate. The bottom (glycerol) layer was removed. The top layer was filtered to afford a pale yellow oil (227 g). ¹H NMR (300 MHz, CDCl₃) δ 4.09 (t, 3H), 3.68 (q, 2H), 2.27 (t, 2H), 1.60 (m, 2H), 1.24 (s, 16H), 0.86 (t, 3H).

### Comparative Example 1 (b)

### Preparation of Dimethylaminopropyl Cocoate

To a 50 mL conical bottom plastic vial was added ethyl cocoate (10 g, 38.5 mmol), dimethylaminopropanol (4.76 g, 46.2 mmol, 1.2 eq) and Novozym™ 435 (400 mg). A syringe was inserted through the cap and two additional holes were punched for gas to exit. Nitrogen was bubbled at a rate sufficient to mix the contents. The vial was placed in a heating block set to 65 °C. The reaction was monitored by GC/MS to observe the disappearance of starting material. The reaction was complete after approximately 24 hours. The reaction mixture was allowed to cool. The Novozym™ 435 was removed by filtration to afford the product as a pale yellow oil (9.2 g) without further purification. ¹H NMR (300 MHz, CDCl₃) δ 4.10 (t, 2H), 2.30 (m, 4H), 2.21 (s, 6H), 1.78 (t, 2H), 1.60 (m, 2H), 1.24 (s, 16H), 0.86 (t, 3H).

### Comparative Example 1(c)

### Preparation of Dimethylaminopropyl Cocoate Betaine

To a 100 mL round bottom flask with a magnetic stir bar and a condenser was added dimethylaminopropyl cocoate (10 g, 35.3 mmol, 1 eq), sodium chloroacetate (4.11 g, 35.3 mmol, 1 eq) and isopropanol (15 mL). The reaction mixture was heated at reflux for 8 hours. When the reaction was complete by NMR, the mixture was allowed to cool. The mixture was filtered and isopropanol was removed *in vacuo* to afford the product as a viscous, semi-solid (14 g). ¹H NMR (300 MHz, CDCl₃) δ 4.16 (t, 2H), 3.92 (t, 2H), 3.67 (t, 2H), 3.28 (s, 6H), 2.34 (q, 2H), 2.10 (t, 2H), 1.60 (m, 2H), 1.26 (s, 16H), 0.88 (t, 3H).

### Example 1(a)

### Preparation of 3-Dimethylaminopropyl Hydroaenated Stripper Cocoate

Hydrogenated and stripped coconut fatty acids (C₁₀-C₁₈ saturated fatty acid mixture) (375 g; 1.69 mol), 3-dimethylaminopropanol (209 g; 2.03 mol; 1.2 equiv), Novozym™ 435 (20 g), and heptane (173 mL) were combined and heated to 65°C. The heptane azeotrope was utilized to remove water by reducing the pressure until the azeotrope distilled overhead into a Dean-Stark trap to return the heptane to the reaction vessel. The reaction was allowed to proceed for 8h at which point GC analysis indicated 99.6% conversion of the hydrogenated stripped coconut fatty acids to the 3-dimethylaminopropyl esters. The enzyme was removed by filtration and the filtrate was concentrated, and the concentrate was purged with nitrogen overnight at 60°C to remove excess 3-dimethylaminopropanol, 99% yield. ¹H NMR (300 MHz, CDCl₃) δ 4.10 (t, 2H), 2.33 (t, 2H), 2.28 (t, 2H); 2.20 (s, 6H); 1.79 (m(5), 2H); 1.60 (m, 2H), 1.24 (m, 16H), 0.86 (t, 3H).

### Example 1(b)

### Preparation of 3-Dimethylaminopropyl Hydroaenated Striped Cocoate Betaines

Sodium chloroacetate (6.53 g; 56.0 mmol; 1.15 equiv), sodium bicarbonate (0.81 g; 9.6 mmol; 0.2 equiv) and 3-dimethylaminopropyl hydrogenated stripped cocoate (15 g; 48.6 mol) were combined in a 100-mL round bottom flask with 33.8 g of water. The mixture was stirred and heated to at 80°C for 13h, at which point HPLC analysis indicated 99.3% conversion to product. The mixture was cooled to ambient temperature and filtered to afford 55.88 g of a 32 wt% (by HPLC) solution of 3-dimethylaminopropyl hydrogenated stripped cocoate betaine in water (99% yield). ¹H NMR (300 MHz, DMSO-d₆) δ 4.03 (t, 2H), 3.58 (s, 2H); 3.10 (s, 6H), 2.27 (t, 2H), 1.96 (m, 2H), 1.49 (m, 2H), 1.22 (m, 16H), 0.83 (t, 3H).

### Example 2(a)

### Preparation of 3-Dimethylaminopropyl Laurate

Lauric acid (600 g; 3.0 mol), 3-dimethylaminopropanol (371 g; 3.59 mol; 1.2 equiv), Novozym™ 435 (30 g), and heptane (267 mL) were combined and heated to 65°C. The heptane azeotrope was utilized to remove water by reducing the pressure until the azeotrope distilled overhead into a Dean-Stark trap to return the heptane to the reaction vessel. The reaction was allowed to proceed for 12h at which point GC analysis indicated 99.3% conversion of lauric acid to to the 3-dimethylaminopropyl ester. The enzyme was removed by filtration and the filtrate was concentrated, and the concentrate was purged with nitrogen overnight at 60°C to remove excess 3-dimethylaminopropanol. ¹H NMR (300 MHz, CDCl₃) δ 4.09 (t, 2H), 2.32 (t, 2H), 2.27 (t, 2H); 2.20 (s, 6H); 1.78 (m(5), 2H); 1.59 (m, 2H), 1.26 (m, 16H), 0.86 (t, 3H).

### Example 2(b)

### Preparation of 3-Dimethylaminopropyl Laurate Betaine

Sodium chloroacetate (292 g; 2.5 mol; 1.1 equiv) and sodium bicarbonate (38.3 g; 0.455 mol; 0.2 equiv) were added to a jacketed 3-L reactor with a mechanical stirrer and a condenser. Water (219 mL), isopropanol (876 mL), and 3-dimethylaminopropyl laurate (650 g; 2.28 mol) were added and the mixture was stirred and the jacket was heated at 81 °C overnight, at which point HPLC analysis indicated 99.6% conversion to product. The mixture was cooled to ambient temperature and 876 mL of isopropanol was added to afford a precipitate. The mixture was filtered and the filtrate was concentrated at reduced pressure. Water (1000 mL) was added, and the mixture was heated to 80°C with a headspace nitrogen purge with periodic addition of water to remove residual isopropanol. Once the isopropanol had been evaporated (¹H NMR analysis), the mixture was cooled to ambient temperature and the pH was adjusted to 6.75 by the addition of 3 M HCl. The resulting mixture was clarified through a scintered glass funnel to afford 2100 g of a 33.0 wt% (by wt% ¹H NMR) solution of 3-dimethylaminopropyl laurate betaine in water (89% yield). ¹H NMR (300 MHz, DMSO-d₆) δ 4.03 (t, 2H), 3.58 (s, 2H); 3.10 (s, 6H), 2.27 (t, 2H), 1.96 (m, 2H), 1.49 (m, 2H), 1.22 (m, 16H), 0.83 (t, 3H).

### Example 3(a)

### Preparation of 3-Dimethylaminopropyl Myristate

Myristic acid (10 g; 43.8 mmol), 3-dimethylaminopropanol (5.87 g; 56.9 mmol; 1.3 equiv), and Novozym™ 435 (2 g) were combined and heated to 65°C with nitrogen sparging at 100 mL/min. After 12h, GC analysis indicated 93.7% conversion of myrstic acid and 100% conversion of dimethylaminopropanol. The enzyme was removed by filtration and the filter cake was washed with heptane. The filtrate was washed with 1:1 methanol:10% aqueous potassium carbonate (30 mL), then with 5% sodium bicarbonate (15 mL), dried with sodium sulfate, and concentrated to afford 12.09 g (88%) of 3-dimethylaminopropyl myristate. ¹H NMR (300 MHz, CDCl₃) δ 4.11 (t, 2H), 2.33 (t, 2H), 2.29 (t, 2H); 2.22 (s, 6H); 1.79 (m(5), 2H); 1.61 (m, 2H), 1.25 (m, 20H), 0.88 (t, 3H).

### Example 3(b)

### Reparation of 3-Dimethylaminopropyl Myristate Betaine

3-Dimethylaminopropyl myristate (5.0; g; 15.95 mmol), sodium chloroacetate (2.04 g; 17.54 mmol; 1.1 equiv) and sodium bicarbonate (268 mg; 3.19 mol; 0.2 equiv) were added to a 100-mL round bottom flask. Water (5 mL) and isopropanol (5 mL) were added, and the mixture was stirred and heated to 80°C for 16h, at which point HPLC analysis indicated 99.1 % conversion. The mixture was cooled to ambient temperature to afford a total solution weight of 15.18 g, indicating approximately 37 wt% 3-dimethylaminopropyl myristate betaine in isopropanol/water. ¹H NMR (300 MHz, DMSO-d₆) δ 4.02 (t, 2H), 3.59 (s, 2H); 3.08 (s, 6H), 2.26 (t, 2H), 1.95 (m, 2H), 1.47 (m, 2H), 1.22 (m, 20H), 0.81 (t, 3H).

### Example 4(a)

### Preparation of 3-Dimethylaminopropyl Cocoate

Coconut fatty acid (32.8 g; 0.154 mol), 3-dimethylaminopropanol (18.15 g; 0.176 mol; 1.14 equiv), Novozym™ 435 (2.62 g), and heptane (15.3 mL) were combined and heated to 50°C. The heptane azeotrope was utilized to remove water by reducing the pressure until the azeotrope distilled overhead into a Dean-Stark trap to return the heptane to the reaction vessel. After 6h GC analysis indicated 99.0% conversion to the 3-dimethylaminopropyl ester of coconut oil fatty acids. The enzyme was removed by filtration and the filtrate was concentrated to afford 41.64 g (90%) of 3-dimethylaminopropyl cocoate.

### Example 4(b)

### Preparation of Dimethylaminopropyl Cocoate Betaine

To a 40 mL vial with a magnetic stir bar and a condenser was added 3-dimethylaminopropyl cocoate (3 g, 10.0 mmol), sodium chloroacetate (1.35 g, 11.6 mmol, 1.15 eq) and sodium bicarbonate (169 mg; 2.0 mmol; 0.2 equiv). Water (6.82 g) was added and the reaction mixture was heated at 80°C for 20 hours at which point HPLC analysis indicated 99.0% conversion to the betaine. The reaction mixture was cooled to afford 10.77g of the product as a 33% solution in water. ¹H NMR (300 MHz, CDCl₃) δ 4.16 (t, 2H), 3.92 (t, 2H), 3.67 (t, 2H), 3.28 (s, 6H), 2.34 (q, 2H), 2.10 (t, 2H), 1.60 (m, 2H), 1.26 (s, 16H), 0.88 (t, 3H). HPLC (150x4.6mm Zorbax SB-C8 column, 80:20 methanol:water (containing 0.1% trifluoroacetic acid) for 10 min, gradient to 100% methanol over 1 min, held at 100% methanol for 9 min, ELSD detection): t_{R} (laurate ester) 3.4 min.

### Example 5(a)

### Preparation of 3-Dimethylaminopropyl Cocoate

Coconut fatty acid (32.8 g; 0.154 mol), 3-dimethylaminopropanol (18.15 g; 0.176 mol; 1.14 equiv), were added to 1080 cm² of *Candida antarctica* lipase B immobilized on a porous fluoropolymer support as described in US Patent Pub. 20120040395. Heptane (30 mL) was added and the mixture was heated to 50°C. The heptane azeotrope was utilized to remove water by reducing the pressure until the azeotrope distilled overhead into a Dean-Stark trap to return the heptane to the reaction vessel. After 6.5h GC analysis indicated 99.9% conversion to the 3-dimethylaminopropyl ester of coconut oil fatty acids. The product solution was decanted and the enzyme was washed with heptane. The combined organic solution was concentrated to remove volatiles and afford 43.49 g (94%) of 3-dimethylaminopropyl cocoate.

### Example 5(b)

### Preparation of Dimethylaminopropyl Cocoate Betaine

To a 40 mL vial with a magnetic stir bar and a condenser was added 3-dimethylaminopropyl cocoate (3 g, 10.0 mmol), sodium chloroacetate (1.35 g, 11.6 mmol, 1.15 eq) and sodium bicarbonate (169 mg; 2.0 mmol; 0.2 equiv). Water (6.82 g) was added and the reaction mixture was heated at 80°C for 20 hours at which point HPLC analysis indicated 99.2% conversion to the betaine. The reaction mixture was cooled to afford 10.97 of the product as a 32% solution in water. ¹H NMR (300 MHz, CDCl₃) δ 4.16 (t, 2H), 3.92 (t, 2H), 3.67 (t, 2H), 3.28 (s, 6H), 2.34 (q, 2H), 2.10 (t, 2H), 1.60 (m, 2H), 1.26 (s, 16H), 0.88 (t, 3H). HPLC (150x4.6mm Zorbax SB-C8 column, 80:20 methanol:water (containing 0.1% trifluoroacetic acid) for 10 min, gradient to 100% methanol over 1 min, held at 100% methanol for 9 min, ELSD detection): t_{R} (laurate ester) 3.4 min.

### Example 6(a)

### Preparation of Dimethylaminoethyl Mysterate

Myristic acid (35.17g; 0.154 mol), 3-dimethylaminopropanol (18.15 g; 0.176 mol; 1.14 equiv), Novozym™ 435 (2.62 g), and heptane (15.3 mL) were combined and heated to 50oC. The heptane azeotrope was utilized to remove water by reducing the pressure until the azeotrope distilled overhead into a Dean-Stark trap to return the heptane to the reaction vessel. After 8h GC analysis indicated 98.5% conversion to 3-dimethylaminopropyl myristate.

### Example 7(a)

### Preparation of 3-Dimethylaminopropyl Cocoate

Coconut fatty acid (32.8 g; 0.154 mol), 3-dimethylaminopropanol (18.15 g; 0.176 mol; 1.14 equiv), Novozym™ 435 (2.62 g), were combined and heated to 50°C. Stirring was started and a nitrogen sparge (500 mL/min) was started. After 8h GC analysis indicated 91.2% conversion to the 3-dimethylaminopropyl ester of coconut oil fatty acids with 3-dimethylaminopropanol still remaining. An additional 0.25 equiv of 3-dimethylaminopropanol (4.0 g; 0.039 mmol) was added and the reaction was continued for an additional 8h, at which point GC analysis indicated 95.8% conversion. An additional 0.25 equiv of 3-dimethylaminopropanol (4.0 g; 0.039 mmol) was added and the reaction was continued for an additional 6h, at which point GC analysis indicated 96.7% conversion. The enzyme was removed by filtration and the filtrate was washed with heptane. The combined organic solution was washed with a mixture of 10% aqueous potassium carbonate (25 mL), methanol (25 mL), and water (20 mL). The layers were separated and the top organic layer was concentrated. The residue was dissolved in heptane, dried with sodium sulfate and the volatiles were removed to afford 40.30 g (87%) of 3-dimethylaminopropyl cocoate.

A comparing of Examples 6(a) to 3(a) and Examples 7(a) to 4(a) show the improvement achieved using an azeotroping agent to remove water. In Example 3(a), without a solvent, conversion of mysteric acid to 3-Dimethylaminopropyl Myristate was after 12 hours was 93.7%, while in Example 6(a), with a solvent the conversion after 8 hours was 98.5%. Likewise, in Example 7(a), without a solvent, conversion to 3-Dimethylaminoprpyl Cocoate after 8 hours was 91.2% while in Example 4(a), with solvent, the conversion after 6 hours was 99.0%

### Comparative Example 2(a)

### Preparation of Dimethylaminoethyl Laurate

Lauric acid (4.66 g; 23.3 mol), dimethylaminoethanol (1.04 g, 11.6 mmol; 0.5 equiv) and *Candida antarctica* lipase B immobilized on an acrylic resin particle (230 mg) (made in the laboratory whereas in other examples purchased Novozym™ 435 was used) were combined and heated to 65°C with a nitrogen sparge to remove the water byproduct. At 2h, 4h, and 6h each an additional 0.50 equiv of dimethylaminoethanol (0.52 g; 5.8 mmol) was added, to afford a total of 2.0 equivalents of dimethylaminoethanol. The reaction was allowed to proceed for a total of 23h at which point GC analysis indicated 84.9% conversion of lauric acid to the dimethylaminoethyl ester. An additional 0.5 equiv of dimethylaminoethanol was added (0.52g; 5.8 mmol) and heating was continued. After 5 additional hours (total 29 h) the conversion was 85.8%, indicating that the reaction had stalled.

Comparative Example 2(a) illustrates a conversion 85.8% after a total of 29 hours when chemical moiety A of formula **1** has only 2 carbon atoms. It is therefore preferred to require at least 3 carbon atoms in moiety A of formula 1, so as to ensure good conversion rates in the invention process.

The disclosed dialkylaminoalkyl cocoate betaines can be advantageously prepared in high yield and purity by a two-step direct esterficiation chemoenzymatic process. These betaine esters are useful as surfactants and in cosmetic formulations.

## Claims

1. A method for the preparation of a betaine represented by general formula 1, 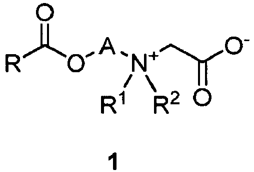 comprising:
a) reacting a carboxylic acid 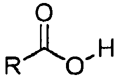 wherein R selected from the group consisting of C₅-C₁₇ alkyl, C₅-C₁₇ alkenyl, C₅-C₁₇ dienyl, C₅-C₁₇ trienyl, and mixtures thereof with a dialkylamino alcohol 3: 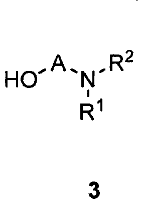 in the presence of an enzyme to form an intermediate **4:** 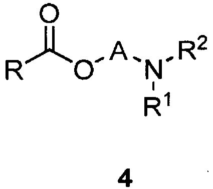 wherein R¹ is methyl and R² is selected from the group consisting of C₁-C₅ alkyl;
A is selected from the group consisting of C₃-C₁₀ alkylene and C₃-C₁₀ alkenylene;
and
b) reacting intermediate **4** with sodium chloroacetate to produce a betaine;
wherein the enzyme is Candida antarctica lipase B immobilized on acrylic resin or Candida antarctica lipase B immobilized on a porous fluoropolymer.

2. The method according to claim 1, wherein R is selected from the group consisting of C₅-C₁₇ alkyl.

3. The method according to any of claims 1-2, wherein R² is methyl.

4. The method according to any of claims 1-3, wherein A is 1,3-propylene.

5. The method according to any of claims 1-4, wherein step (a) of the direct esterification process is carried out without a solvent.

6. The method of any of claims 1-4 wherein step (a) of the direct esterification process is carried out in an inert solvent.

7. The method of claim 6 wherein the inert solvent is chosen from: cyclic or acyclic ether solvents such as diethyl ether, diisopropyl ether, tert-butyl methyl ether, or tetrahydrofuran, aromatic hydrocarbons such as benzene, toluene, or xylene, aliphatic or alicyclic saturated or unsaturated hydrocarbons such as hexane, heptane, cyclohexane, or limonene, halogenated hydrocarbons such as dichloromethane, dichloroethane, dibromoethane, tetrachloroethylene, or chlorobenzene, polar aprotic solvents such as acetonitrile, dimethyl formamide, or dimethyl sulfoxide, or mixtures thereof.

8. The method of claim 7 wherein the inert solvent is an azeotroping agent, preferably heptane.

9. The method according to any of claims 1-8 wherein the betaine ester is incorporated into a product formulation in an amount of from 0.001 wt% to 20 wt%.

10. The method according to claim 9 wherein the amount of betaine ester is 0.1 wt% to 10 wt%.

11. The method of claim 9 or claim 10 wherein the product formulation is selected from a cosmetic, skin, and hair care composition.

## Patentansprüche

1. Verfahren zur Herstellung eines Betains, dargestellt durch die allgemeine Formel **1,** 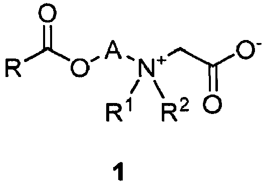 umfassend:
a) Umsetzung einer Carbonsäure 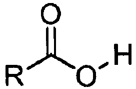 wobei R ausgewählt aus der Gruppe bestehend aus C₅-C₁₇-Alkyl, C₅-C₁₇-Alkenyl, C₅-C₁₇-Dienyl, C₅-C₁₇-Trienyl und Mischungen davon mit einem Dialkylaminoalkohol **3:** 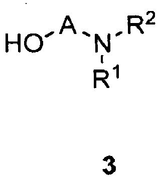 in Gegenwart eines Enzyms, um ein Zwischenprodukt 4 zu bilden: 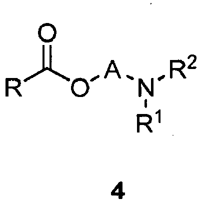 wobei R¹ Methyl ist und R² ausgewählt ist aus der Gruppe bestehend aus C₁-C₅-Alkyl;
A ausgewählt ist aus der Gruppe bestehend aus C₃-C₁₀-Alkylen und C₃-C₁₀-Alkenylen;
und
b) Umsetzen von Zwischenprodukt **4** mit Natriumchloracetat zur Herstellung eines Betains;
wobei das Enzym Candida-Antarktis-Lipase B ist, die auf Acrylharz immobilisiert ist oder Candida-Antarktis-Lipase B ist, die auf einem porösen Fluorpolymer immobilisiert ist.

2. Verfahren nach Anspruch 1, wobei R ausgewählt ist aus der Gruppe bestehend aus C₅-C₁₇-Alkyl.

3. Verfahren nach einem der Ansprüche 1 bis 2, wobei R² für Methyl steht.

4. Verfahren nach einem der Ansprüche 1 bis 3, wobei A für 1,3-Propylen steht.

5. Verfahren nach einem der Ansprüche 1 bis 4, wobei der Schritt (a) des Direktveresterungsprozesses ohne Lösungsmittel durchgeführt wird.

6. Verfahren nach einem der Ansprüche 1 bis 4, wobei der Schritt (a) des Direktveresterungsprozesses in einem inerten Lösungsmittel durchgeführt wird.

7. Verfahren nach Anspruch 6, wobei das inerte Lösungsmittel ausgewählt ist aus: cyclische oder acyclische Etherlösungsmittel wie Diethylether, Diisopropylether, tert.-Butylmethylether oder Tetrahydrofuran, aromatische Kohlenwasserstoffe wie Benzol, Toluol oder Xylol, aliphatische oder alicyclische gesättigte oder ungesättigte Kohlenwasserstoffe wie Hexan, Heptan, Cyclohexan oder Limonen, halogenierte Kohlenwasserstoffe wie Dichlormethan, Dichlorethan, Dibromethan, Tetrachlorethylen oder Chlorbenzol, polare aprotische Lösungsmittel wie Acetonitril, Dimethylformamid oder Dimethylsulfoxid oder Mischungen davon.

8. Verfahren nach Anspruch 7, wobei das inerte Lösungsmittel ein Azeotropiermittel ist, vorzugsweise Heptan.

9. Verfahren nach einem der Ansprüche 1 bis 8, wobei der Betainester in einer Produktformulierung in einer Menge von 0,001 Gew.-% bis 20 Gew.-% eingearbeitet wird.

10. Verfahren nach Anspruch 9, wobei die Menge an Betainester 0,1 Gew.-% bis 10 Gew.-% beträgt.

11. Verfahren nach Anspruch 9 oder 10, wobei die Produktformulierung aus einer kosmetischen Zusammensetzung, einer Hautzusammensetzung und einer Haarpflegezusammensetzung ausgewählt ist.

## Revendications

1. Méthode de préparation d'une bétaïne représentée par la formule générale 1, 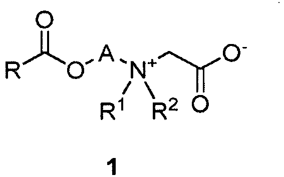 comprenant :
a) la réaction d'un acide carboxylique 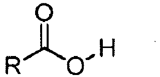 dans lequel R est choisi dans le groupe constitué par un alkyle en C₅-C₁₇, un alcényle en C₅-C₁₇, un diényle en C₅-C₁₇, un triényle en C₅-C₁₇, et leurs mélanges avec un alcool dialkylamino **3 :** 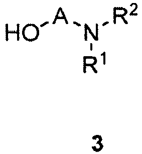 en présence d'une enzyme pour former un intermédiaire **4 :** 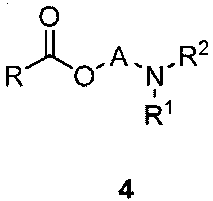 dans lequel R¹ est un méthyle et R² est choisi dans le groupe constitué par un alkyle en C₁-C₅ ;
A est choisi dans le groupe constitué par un alkylène en C₃-C₁₀ et un alcénylène en C₃-C₁₀ ; et
b) la réaction de l'intermédiaire **4** avec un chloroacétate de sodium pour obtenir une bétaïne,
dans laquelle l'enzyme est la lipase B de Candida antarctica immobilisée sur une résine acrylique ou la lipase B de Candida antarctica immobilisée sur un polymère fluoré poreux.

2. Méthode selon la revendication 1, dans laquelle R est choisi dans le groupe constitué par un alkyle en C₅-C₁₇.

3. Méthode selon l'une quelconque des revendications 1-2, dans laquelle R² est un méthyle.

4. Méthode selon l'une quelconque des revendications 1-3, dans laquelle A est le 1,3-propylène.

5. Méthode selon l'une quelconque des revendications 1-4, dans laquelle l'étape (a) du procédé d'estérification directe est mise en oeuvre sans solvant.

6. Méthode selon l'une quelconque des revendications 1-4, dans laquelle l'étape (a) du procédé d'estérification directe est mise en oeuvre dans un solvant inerte.

7. Méthode selon la revendication 6, dans laquelle le solvant inerte est choisi parmi : les solvants de type éther cyclique ou acyclique tels que l'éther diéthylique, l'éther diisopropylique, l'éther tert-butylméthylique, ou le tétrahydrofurane, les hydrocarbures aromatiques tels que le benzène, le toluène, ou le xylène, les hydrocarbures aliphatiques ou alicycliques saturés ou insaturés tels que l'hexane, l'heptane, le cyclohexane, ou le limonène, les hydrocarbures halogénés tels que le dichlorométhane, le dichloroéthane, le dibromoéthane, le tétrachloroéthylène, ou le chlorobenzène, les solvants aprotiques polaires tels que l'acétonitrile, le diméthylformamide, ou le diméthylsulfoxyde, ou leurs mélanges.

8. Méthode selon la revendication 7 dans laquelle le solvant inerte est un agent azéotrope, de préférence l'heptane.

9. Méthode selon l'une quelconque des revendications 1-8 dans laquelle l'ester de bétaïne est incorporé dans une formulation de produit en une quantité 0,001 à 20 % en poids.

10. Méthode selon la revendication 9 dans laquelle la quantité d'ester de bétaïne est de 0,1 à 10 % en poids.

11. Méthode selon la revendication 9 ou la revendication 10 dans laquelle la formulation de produit est choisie parmi une composition cosmétique, une composition de soins pour la peau, et pour les cheveux.
